# EUROPÄISCHE PATENTSCHRIFT

(11) **EP 1 248 599 B1**
(45) Veröffentlichungstag und Bekanntmachung des Hinweises auf die Patenterteilung: **05.12.2007**
(21) Anmeldenummer: 01933676.7
(22) Anmeldetag: 09.03.2001
(51) Int. Cl.: A61K 9/26

(54) **MULTIPARTIKULÄRE ARZNEIFORM, ENTHALTEND MINDESTENS ZWEI UNTERSCHIEDLICH ÜBERZOGENE PELLETFORMEN**
MULTI-PARTICULATE FORM OF MEDICAMENT, COMPRISING AT LEAST TWO DIFFERENTLY COATED FORMS OF PELLET
FORME GALENIQUE MULTIPARTICULAIRE CONTENANT AU MOINS DEUX FORMES DE PELLETS ENROBES DE DIFFERENTES MANIERES

(30) Priorität: 31.01.2001 DE 10104504; 01.02.2001 DE 10104880
(43) Veröffentlichungstag der Anmeldung: 16.10.2002
(73) Patentinhaber: Evonik Röhm GmbH, 64293 Darmstadt (DE)
(72) Erfinder: BECKERT, Thomas, 88447 Warthausen (DE); PETEREIT, Hans-Ulrich, 64291 Darmstadt (DE); DRESSMAN, Jennifer, 60322 Frankfurt (DE); RUDOLPH, Markus, 63263 Neu-Isenburg (DE)
(74) Vertreter: Gottschalk, Michael
(86) Internationale Anmeldenummer: PCT/EP2001/002679
(87) Internationale Veröffentlichungsnummer: WO 2002/060415

(56) Entgegenhaltungen:
- US-A- 4 600 577
- GUPTA V K ET AL: "Statistical optimization of a novel multi-unit colonic delivery system containing multiple coatings of aqueous polymethacrylates" 27TH INTERNATIONAL SYMPOSIUM ON CONTROLLED RELEASE OF BIOACTIVE MATERIALS AND 3RD CONSUMER AND DIVERSIFIED PRODUCTS CONFERENCE. PROCEEDINGS BOOK 2000. PARIS, FRANCE, JULY 7 - 13, 2000, DEERFIELD, IL: CRS, US, 7. Juli 2000 (2000-07-07), Seiten 453-454, XP002172814
- DATABASE BIOSIS [Online] BIOSCIENCES INFORMATION SERVICE, PHILADELPHIA, PA, US; Dezember 2000 (2000-12) FU CHONG-DONG ET AL: "Preparation and evaluation of pH-dependent and sustained-release pellets for mesalazine colon targeted delivery." Database accession no. PREV200100122930 XP002192585 & ZHONGGUO YIYAO GONGYE ZAZHI, Bd. 31, Nr. 12, Dezember 2000 (2000-12), Seiten 541-544, ISSN: 1001-8255

## Beschreibung

Die Erfindung betrifft eine multipartikuläre Arzneiform, die mindestens zwei unterschiedlich überzogene Pelletformen enthält und eine weitgehend gleichmäßige Wirkstofffreisetzung über den gesamten Darmbereich ermöglicht. Die Erfindung betrifft weiterhin ein Verfahren zur Herstellung der multipartikulären Arzneiform sowie die Verwendung der Pelletformen A und B zur Herstellung der Arzneiform

### Stand der Technik

Multipartikuläre Arzneiformen, die durch Verpressen eines Bindemittels mit wirkstoffhaltigen, mit magensaftresistenten (Meth)acrylat Copolymeren überzogenen Pellets erhalten werden, sind aus Beckert et al. (1996), "Compression of enteric-coated pellets to disintegrating tablets", International Journal of Pharmaceutics 143, S. 13 - 23, bekannt.

(Meth)acrylat-Copolymere, die Monomere mit quaternären Ammoniumgruppen, z. B. Trimethylammoniumethlymethacrylat-Chlorid, enthalten und deren Verwendung für retardierende Arzneimittelüberzüge sind seit langem bekannt (z. B. aus EP-A 181 515 oder aus DE-PS 1 617 751). Die Verarbeitung erfolgt in organischer Lösung oder als wäßrige Dispersion z. B. durch Sprühen auf Arzneimittelkerne oder auch ohne Lösungsmittel in Gegenwart von Fließmitteln durch Aufbringen in der Schmelze (s. EP-A 0 727 205).

EP-A 629 398 beschreibt pharmazeutische Formulierungen, die einen Kern mit einem Wirkstoff und einer organischen Säure aufweisen, wobei der Kern zweischichtig umhüllt ist. Die innere Hülle wird dabei von einem retardierenden (Meth)acrylatcopolymer mit quaternären Ammoniumgruppen (EUDRAGIT® RS) gebildet, während die äußere Hülle einen magensaftresitenten Überzug aufweist, beispielweise ein Copolymer von Typ EUDRAGIT® L30D-55 (Ethylacrylat/Methacrylsäure, 50 : 50). Die erzielte Freisetzungscharakteristik kann durch eine zeitverzögerte, rasche Wirkstofffreissetzung bei erhöhten pH-Wert beschrieben werden.

EP 0 704 207 A2 beschreibt thermoplastische Kunststoffe für darmsaftlösliche Arzneiumhüllungen. Es handelt sich dabei um Mischpolymerisate aus 16 bis 40 Gew.-% Acryl- oder Methacrylsäure, 30 bis 80 Gew.-% Methylacrylat und 0 bis 40 Gew.-% anderen Alkylestern der Acrylsäure und/oder Methacrylsäure.

EP 0 704 208 A2 beschreibt Überzugs- und Bindemittel für darmsaftlösliche Arzneiumhüllungen. Es handelt sich dabei um Copolymerisate aus 10 bis 25 Gew.-% Methacrylsäure, 40 bis 70 Gew.-% Methylacrylat und 20 bis 40 Gew-% Methylmethacrylat. Die Beschreibung erwähnt neben einschichtigen Überzügen auch mehrlagige Überzugssysteme. Diese können aus einem Kern, der z. B. einen basischen oder einen wasserempfindlichen Wirkstoff enthält, bestehen, weisen eine Isolierschicht aus einem anderen Überzugsmaterial, wie Celluloseether, Celluloseester oder einem kationischen Polymethacrylat z. B. von Typ EUDRAGIT®, u. a. auch EUDRAGIT® RS und RL, auf und werden dann zusätzlich mit der oben genannten darmsaftlöslichen Umhüllung versehen.

Gupka et al. (27th International Symposium on Controlled Release of Bioactive Materials and 3rd Comsumer and Diversified Products conference, Proceedings Book 2000, Seiten 453-454) beschreiben eine multipartikuläre 5-Aminosalicylsäure enthaltende Arzneiform, deren Partikel einen inneren pH-unabhängigen Überzug mit Eudragit RL und RS, sowie einen äußeren pH-abhängigen Überzug mit Eudragit FS aufweisen.

Multipartikuläre Arzneiformen in Form von Kapseln oder verpreßten Tabletten sind hinlänglich bekannt. Auch ist es bekannt in multipartikulären Arzneiformen Pellets mit verschiedenen Polymerüberzügen einzubringen, um so zu kombinierten Freisetzungprofilen zu gelangen.

### Aufgabe und Lösung

Es besteht ein Bedarf an Arzneiformen, die Wirkstoffe im Darmtrakt freisetzen und dabei spezielle Wirkstofffreisetzungsprofile erfüllen.

Es sollte eine Arzneiform bereitgestellt werden, die im Magen nahezu keinen Wirkstoff abgibt und eine möglichst gleichmäßige und langanhaltende Wirkstoffabgabe sowohl im Dünndarm als auch im Dickdarmbereich ermöglicht. Die Arzneiform soll z. B. geeignet sein zur Therapie von entzündlichen Darmerkrankungen wie Colitis ulcerosa und insbesondere Morbus Crohn.

Die Aufgabe wird gelöst durch eine
Multipartikuläre Arzneiform, geeignet zur gleichmäßigen Freisetzung eines pharmazeutischen Wirkstoffs im Dünndarm und im Dickdarm, enthaltend mindestens zwei Formen von Pellets A und B, die im Kern einen pharmazeutischen Wirkstoff enthalten und unterschiedliche Polymerüberzüge aufweisen, die die Freisetzung des Wirkstoffs bei unterschiedlichen pH-Werten bestimmen, dadurch gekennzeichnet, dass
die Pelletform A mit einem inneren Polymerüberzug versehen ist, der eine kontinuierliche Wirkstofffreigabe ermöglicht, und einen äußeren magensaftresistenten Überzug aufweist, der sich oberhalb von etwa pH 5,5 schnell auflöst und der aus einem (Meth)acrylat-Copolymer aus 40 bis 60 Gew.-% Methacrylsäure und 60 bis 40 Gew.-% Methylmethacrylat oder 60 bis 40 Gew.-% Ethylacrylat, oder HPMCP (Hydroxypropylmethylcellulosephthalat), besteht und
die Pelletform B mit einem Polymerüberzüg versehen ist, der im Freisetzungstest nach USP bei pH 6,8 in 6 Stunden weniger als 20 % des Wirkstoff freisetzt und bei pH 7,2 in 6 Stunden mehr als 50 % des Wirkstoffs freisetzt, wobei der Polymerüberzug der Pelletform B ein (Meth)acrylat-Copolymer ist, das sich aus 60 bis 95 Gew.-% radikalisch-polymerisierten C₁- bis C₄-Alkylestern der Acryl- oder der Methacrylsäure und 5 bis 40 Gew.% (Meth)acrylat-Monomeren mit einer Säuregruppe im Alkylrest zusammensetzt.

Die Erfindung betrifft weiterhin ein Verfahren zur Herstellung einer multipartikulären Arzneiform, indem man die unterschiedlichen Pelletformen A und B mittels Überziehens von wirkstoffhaltigen Kernen mit den angegebenen Polymerüberzügen herstellt, miteinander mischt und durch Einfüllen in eine Kapsel oder Verpressen zu einer Tabletteneinheit in Gegenwart von Hilfsstoffen in eine multipartikuläre Arzneiform überführt.

Die Erfindung betrifft ebenso die Verwendung der beschriebenen Pelletformen A und B im beanspruchten Verfahren zur Herstellung einer multipartikulären Arzneiform mit einer gleichmäßigen Wirkstoffabgabe im pH-Bereich von 6,8 und 7,2, entsprechend den Verhältnissen in Dünn- und Dickdarm, insbesondere zur Behandlung von Morbus Crohn oder Colitis ulcerosa.

### Ausführung der Erfindung

Die Multipartikuläre Arzneiform kann in Form einer mit Pellets gefüllten Kapsel, z. B. einer Gelatinekapsel, vorliegen oder es kann sich um eine Tablette handeln, in der die Pellets zusammen mit üblichen Hilfsstoffen zur Tabletteneinheit verpreßt wurden.

Die Multipartikuläre Arzneiform ist geeignet zur weitgehend gleichmäßigen Freisetzung eines pharmazeutischen Wirkstoffs im Dünndarm und im Dickdarm und enthält mindestens zwei Formen von Pellets, A und B, die im Kern einen pharmazeutischen Wirkstoff enthalten, aber unterschiedliche Polymerüberzüge aufweisen, die die Freisetzung des Wirkstoffs bei unterschiedlichen pH-Werten bestimmen. In vitro erhält man im Freisetzungstest nach USP (USP 23, Methode 2) bei pH 6,8 und bei pH 7,2 Mischprofile die zwischen den individuellen Freisetzungskurven der beiden Pelletformen A und B liegen. In vivo dominiert im Dünndarm das Freisetzungsprofil der Pelletform A und während im Dickdarmbereich die Wirkstoffreigabe aus dem Pelletform B einsetzt.

Die Pelletkerne bestehen ganz oder teilweise aus einem pharmazeutischen Wirkstoff. Die Kerne sind in der Regel sphärisch oder rund und haben Durchmesser im Bereich von etwa 0,3 bis 2 mm. Die Polymerüberzüge liegen im Bereich von etwa 2 bis 16 mg Polymer pro cm² Oberfläche der Kerne.

### Pelletform A

Die Pelletform A ist mit einen inneren Polymerüberzug und einem äußeren Polymerüberzug versehen.

### Innerer Polymerüberzug

Der innere Polymerüberzug ermöglicht eine weitgehend pH-unabhängige kontinuierliche Wirkstofffreigabe. Es wird ein Wirkstofffreigabeprofil angestrebt, bei dem im Freisetzungstest nach USP (USP 23, Methode 2) bei pH 6,8 nach 2 Stunden etwa 40 bis 70 %, bevorzugt 40 bis 60 % und nach 4 Stunden 60 bis 100 %, bevorzugt 80 bis 100 % des Wirkstoffs freigesetzt werden. Dies leitet sich von der durchschnittlichen Verweildauer im Dünndarm, die etwa 4 Stunden beträgt ab.

Der innere Polymerüberzug der Pelletform A kann aus einem (Meth)acrylat-Copolymer, aus radikalisch polymerisierten C1- bis C4-Alkylestern der Acryl- oder der Methacrylsäure und (Meth)acrylat-Monomeren mit einer quaternären Ammoniumgruppe im Alkylrest bestehen.

Entsprechende (Meth)acrylat-Copolymere sind z. B. aus EP-A 181 515 oder aus DE-PS 1 617 751 bekannt. Es handelt sich um unabhängig vom pH-Wert lösliche oder quellbare Polymerisate, die für Arzneimittelüberzügen geeignet sind. Als mögliches Herstellungverfahren ist die Substanzpolymeriation in Gegenwart eines im Monomerengemisch gelösten radikalbildenden Initiators zu nennen. Ebenso kann das Polymerisat auch mittels Lösungs- oder Fällungspolymerisation hergestellt werden. Das Polymerisat kann auf diese Weise in Form eines feinen Pulvers erhalten werden, was bei der Subtanzpolymerisation durch Mahlen, bei Lösungs- und Fällungspolymerisation z. B. durch Sprühtrocknung erreichbar ist.

Das (Meth)acrylat-Copolymer, setzt sich aus 85 bis 98 Gew.-% radikalisch polymerisierten C1- bis C4-Alkylestern der Acryl- oder der Methacrylsäure und 15 bis 2 Gew.-% (Meth)acrylat-Monomeren mit einer quaternären Ammoniumgruppe im Alkylrest zusammen.

Bevorzugte C1- bis C4-Alkylestern der Acryl- oder der Methacrylsäure sind Methylacrylat, Ethylacrylat, Butylacrylat, Butylmethacrylat und Methylmethacrylat.

Als (Meth)acrylat Monomer mit quaternären Ammoniumgruppen wird 2-Trimethylammoniumethylmethacrylat-Chlorid besonders bevorzugt.

Ein weiteres geeignetes (Meth)acrylat-Copolymer kann z. B. aus 85 bis weniger als 93 Gew.-% C1- bis C4-Alkylestern der Acryl- oder der Methacrylsäure und mehr als 7 bis 15 Gew.-% (Meth)acrylat Monomeren mit einer quaternären Ammoniumgruppe im Alkylrest aufgebaut sein. Derartige (Meth)acrylatMonomere sind handelsüblich und werden seit langem für retardierende Überzüge verwendet (Typ (EUDRAGIT® RL).

Ein konkret geeignetes Copolymer enthält z. B. 60 Gew.-% Methylmethacrylat, 30 Gew.-% Ethylacrylat und 10 Gew.-% 2-Trimethylammoniumethlymethacrylat-Chlorid (EUDRAGIT® RL).

Die gewünschte Freisetzungscharakteristik kann z. B. durch die Dicke der Überzugsschicht von Polymerüberzügen vom oben beschriebenen "Typ EUDRAGIT® RL" erreicht werden. Diese wird z. B. bei einem 5 bis 15 %-igem Überzug von EUDRAGIT® RL auf wirkstoffhaltigen Kernen mit 0,8 bis 1,2 mm Durchmesser erreicht. Die gewünschte Freisetzungscharakteristik kann auch bei anderen Schichtdicken durch Zumischen eines Copolymers, aus 50 - 70 Gew.-% Methylmethacrylat, 20 - 40 Gew.-% Ethylacrylat und 7 - 2 Gew.-% 2-Trimethylammoniumethylmethacrylat-Chlorid aufgebaut sein ("Typ EUDRAGIT® RS") erreicht werden. Ein konkret geeignetes Copolymer enthält 65 Gew.-% Methylmethacrylat, 30 Gew.-% Ethylacrylat und 5 Gew.-% 2-Trimethylammoniumethylmethacrylat-Chlorid aufgebaut sein (EUDRAGIT® RS). Die Typen EUDRAGIT® RL und RS können z. B. in den Verhältnissen 10 zu 1 bis 1 zu 10 gemischt werden. Bevorzugt sind höhere Anteile des "EUDRAGIT® RL-Typs", z. B. 60 bis 90 Gew.-% in der Mischung.

Der innere Polymerüberzug kann auch aus einem (Meth)acrylat Copolymeren aus 20 bis 40 Gew.-% Ethylacrylat und 60 bis 80 Gew.-% Methylmethacrylat, Ethylcellulose oder Polyvinylacetat bestehen.

### Äußerer Polymerüberzug

Der äußere Polymerüberzug ist ein magensaftresistenter Überzug, der sich erst oberhalb von etwa pH 5,5 schnell auflöst. Der Überzug soll somit eine Wirkstofffreisetzung im Magen weitgehend verhindern, d. h. diese soll nach USP 23 höchstens 10, bevorzugt nur 5 % betragen. Beim Übergang in den Dünndarm soll sich der äußere Polymerüberzug rasch auflösen, so daß die Freigabecharakteristik ab diesem Zeitpunkt vom inneren Polymerüberzug bestimmt wird. Ist der äußere Polymerüberzug zu dünn, wird bereits im Magen zu viel Wirkstoff freigesetzt. Ist der äußere Polymerüberzug zu dick aufgetragen, behindert er die unmittelbare Wirkstofffreisetzung im Dünndarm. Geeignete Schichtdicken liegen z. B. im Bereich von 15 bis 150 µm, bevorzugt z. B. bei 20 bis 60 µm. Bezogen auf das Gewicht des mit dem inneren Polymerüberzug versehenen Kerns mit einem Durchmesser von 0,8 bis 1,25 mm, ist in der Regel ein Polymerauftrag (bezogen auf Trockensubstanz) im Bereich von 8 bis 40 Gew.-% , bevorzugt von 10 bis 25 Gew.-% geeignet.

Der magensaftresistente Polymerüberzug der Pelletform A kann aus

(Meth)acrylat Copolymen aus 40 bis 60 Gew.-% Methacrylsäure und 60 bis 40 Gew.-% Methylmethacrylat oder 60 bis 40 Gew.-% Ethylacrylat (Typen EUDRAGIT® L oder EUDRAGIT® L100-55) bestehen.

Der magensaftresistente Polymerüberzug der Pelletform A kann auch aus HPMCP (Hydroxypropylmethylcellulosephthalat) bestehen.

In jedem Fall ist jedoch darauf zu achten, daß der Überzug zum Beispiel in Bezug auf Schichtdicke und ggf. Mischung mit anderen Polymeren so eingestellt wird, daß er sich nach Übergang in den Dünndarm zügig auflöst.

### Pelletform B

Die Pelletform B setzt bei pH 6,8 im Freisetzungstest nach USP (USP 23, Methode 2) nach 2 Stunden nicht mehr als 10 %, bevorzugt nicht mehr als 5 % und nach 4 Stunden nicht mehr als 20, bevorzugt nicht mehr als 10 % des Wirkstoffs frei. Bei pH 7, 2 werden nach 3 Stunden etwa 40 bis 60 % und nach 60 Stunden etwa 80 bis 100 des Wirkstoffs freigesetzt.

Der Polymerüberzug für die Pelletform B ist ein (Meth)acrylat-Copolymer das sich aus 60 bis 95 Gew.-% radikalisch polymerisierten C₁- bis C₄-Alkylestern der Acryl- oder der Methacrylsäure und 5 bis 40 Gew.-% (Meth)acrylat-Monomeren mit einer Säuregruppe im Alkylrest zusammensetzt.

Besonders gut geeignet sind (Meth)acrylat Copolymere, bestehend aus 10 bis 30 Gew.-%, Methylmethacrylat, 50 bis 70 Gew.-% Methylacrylat und 5 bis 15 Gew.-% Methacrylsäure (Typ EUDRAGIT® FS).

Ebenso geeignet sind (Meth)acrylat Copolymere aus 20 bis 40 Gew.-% Methacrylsäure und 80 bis 60 Gew.-% Methylmethacrylat (Typ EUDRAGIT® S).

Die Pelletform B ist bevorzugt mit nur einem Polymerüberzug versehen, kann jedoch wenn das Freigabeprofil im Dickdarm modifiziert werden soll, auch wie die Pelletform A zusätzlich mit einem inneren Polymerüberzug versehen werden, der eine weitgehend pH-unabhängige kontinuierliche Wirkstofffreigabe bedingt. Dies kann sinnvoll sein, wenn es erforderlich ist, die Wirkstofffreigabe im Dickdarm (Colon) auf 6 bis 12 oder bis zu 24 Stunden auszudehnen.

### Wirkstoffe

Die erfindungsgemäße Formulierung eignet sich zur Verabreichung einer Vielzahl pharmazeutischer Wirkstoffe, die im Dünndarm und im Dickdarm freigesetzt werden sollen, und insbesondere solcher Wirkstoffe, die mit Vorteil in retardierter Form verabreicht werden können, wie Antidiabetika, Analgetika, Antiphlogistika, Antirheumatika, Antihypotonika, Antihypertonika, Psychopharmaka, Tranquilizer, Antiemetika, Muskelrelaxantien, Glucocorticoide, Mittel zur Behandlung von Colitis ulcerosa oder Morbus Crohn, Antiallergika, Antibiotika, Antiepileptika, Antikoagulantia, Antimykotika, Antitussiva, Arteriosklerosemittel, Diuretika, Proteine, Peptide, Enzyme, Enzyminhibitoren, Gichtmittel, Hormone und deren Hemmstoffe, Herzglykoside, lmmuntherapeutika und Zytokine, Laxantien, Lipidsenker, Migränemittel, Mineralstoffpraparate, Otologika, Parkinsonmittel, Schilddrüsentherapeutika, Spasmolytika, Thrombozytenaggregationshemmer, Vitamine, Zytostatika und Metastasenhemmer, Phytopharmaka, Chemotherapeutika und Aminosäuren.

Beispiele in Frage kommender Wirkstoffe sind Acarbose, Antigene, Betarezeptorenblocker, Nichtsteroidale Antirheumatia, Herzglykoside, Acetylsalicylsäure, Virustatika, Aclarubicin, Acyclovir, Cisplatin, Actinomycin, alpha- und beta-Sympatomimetika, (Dmeprazol, Allopurinol, Alprostadil, Prostaglandine, Amantadin, Ambroxol, Amlodipin, Methotrexat, S-Aminosalicylsäure, Amitriptylin, Amoxicillin, Anastrozol, Atenolol, Azathioprin, Balsalazid, Beclomethason, Betahistin, Bezafibrat, Bicalutamid, Diazepam und Diazepamderivate, Budesonid, Bufexamac, Buprenorphin, Methadon, Calciumsalze, Kaliumsalze, Magnesiumsalze, Candesartan, Carbamazepin, Captopril, Cefalosporine, Cetirizin, Chenodeoxycholsäure, Ursodeoxycholsäure, Theophyllin und Theophyllinderivate, Trypsine, Cimetidin, Clarithromycin, Clavulansäure, Clindamycin, Clobutinol, Clonidin, Cotrimoxazol, Codein, Coffein, Vitamin D und Derivate von Vitamin D, Colestyramin, Cromoglicinsäure, Cumarin und Cumarinderivate, Cystein, Cytarabin, Cyclophosphamid, Ciclosporin, Cyproteron, Cytarabin, Dapiprazol, Desogestrel, Desonid, Dihydralazin, Diltiazem, Mutterkornalkaloide, Dimenhydrinat, Dimethylsulfoxid, Dimeticon, Dipyridarnoi, Domperidon und Domperidanderivate, Dopamin, Doxazosin, Doxorubizin, Doxylamin, Dapiprazol, Benzodiazepine, Diclofenac, Glykosidantibiotika, Desipramin, Econazol, ACE-Hemmer, Enalapril, Ephedrin, Epinephrin, Epoetin und Epoetinderivate, Morphinane, Calciumantagonisten, Irinotecan, Modafinil, Orlistat, Peptidantibiotika, Phenytoin, Riluzole, Risedronat, Sildenafil, Topiramat, Makrolidantibiotika, Estrogen und Estrogenderivate, Gestagen und Gestagenderivate, Testosteron und Testosteronderivate, Androgen und Androgenderivate, Ethenzamid, Etofenamat, Etofibrat, Fenofibrat, Etofyllin, Etoposid, Famciclovir, Famotidin, Felodipin, Fenofibrat, Fentanyl, Fenticonazol, Gyrasehemmer, Fluconazol, Fludarabin, Flunarizin, Fluorouracil, Fluoxetin, Flurbiprofen, Ibuprofen, Flutamid, Fluvastatin, Follitropin, Formoterol, Fosfomicin, Furosemid, Fusidinsäure, Gallopamil, Ganciclovir, Gemfibrozil, Gentamicin, Ginkgo, Johanniskraut, Glibenclamid, Harnstoffderivate als orale Antidiabetika, Glucagon, Glucosamin und Glucosaminderivate, Glutathion, Glycerol und Glycerolderivate, Hypothalamushormone, Goserelin, Gyrasehemmer, Guanethidin, Halofantrin, Haloperidol, Heparin und Heparinderivate, Hyaluronsäure, Hydralazin, Hydrochlorothiazid und Hydrochlorothiazidderivate, Salicylate, Hydroxyzin, ldarubicin, Ifosfamid, Imipramin, Indometacin, Indoramin, Insulin, Interferone, Jod und Jodderivate, Isoconazol, Isoprenalin, Glucitol und Glucitolclerivate, Itraconazol, Ketoconazol, Ketoprofen, Ketotifen, Lacidipin, Lansoprazol, Levodopa, Levomethadon, Schilddrüsenhormone, Liponsäure und Liponsäurederivate, Lisinopril, Lisurid, Lofepramin, Lomustin, Loperamid, Loratadin, Maprotilin, Mebendazol, Mebeverin, Meclozin, Mefenaminsäure, Mefloquin, Meloxicam, Mepindolol, Meprobamat,Meropenem, Mesalazin, Mesuximid, Metamizol, Metformin, Methotrexat, Methylphenidat, Methylprednisolon, Metixen, Metoclopramid, Metoprolol, Metronidazol, Mianserin, Miconazol, Minocyclin, Minoxidil, Misoprostol, Mitomycin, Mizolastin, Moexipril, Morphin und Morphinderivate, Nachtkerze, Nalbuphin, Naloxon, Tilidin, Naproxen, Narcotin, Natamycin, Neostigmin, Nicergolin, Nicethamid, Nifedipin, Nifluminsäure, Nimodipin, Nimorazol, Nimustin, Nisoldipin, Adrenalin und Adrenalinderivate, Norfloxacin, Novaminsulfon, Noscapin, Nystatin, Ofloxacin, Olanzapin, Olsalazin, Omeprazol, Omoconazol, Ondansetron, Oxaceprol, Oxacillin, Oxiconazol, Oxymetazolin, Pantoprazol, Paracetamol, Paroxetin, Penciclovir, orale Penicilline, Pentazocin, Pentifyllin, Pentoxifyllin, Perphenazin, Pethidin, Pflanzenextrakte, Phenazon, Pheniramin, Barbitursäurederivate, Phenylbutazon, Phenytoin, Pimozid, Pindolol, Piperazin, Piracetam, Pirenzepin, Piribedil, Piroxicam, Pramipexol, Pravastatin, Prazosin, Procain, Promazin, Propiverin, Propranolol, Propyphenazon, Prostaglandine, Protionamid, Proxyphyllin, Quetiapin, Quinapril, Quinaprilat, Ramipril, Ranitidin, Reproterol, Reserpin, Ribavirin, Rifampicin, Risperidon, Ritonavir, Ropinirol, Roxatidin, Roxithromycin, Ruscogenin, Rutosid und Rutosidderivate, Sabadilla, Salbutamol, Salmeterol, Scopolamin, Selegilin, Sertaconazol, Sertindol, Sertralion, Silikate, Sildenafil, Simvastatin, Sitosterin, Sotalol, Spagluminsäure, Sparfloxacin, Spectinomycin, Spiramycin, Spirapril, Spironolacton, Stavudin, Streptomycin, Sucralfat, Sufentanil, Sulbactam, Sulfonamide, Sulfasalazin, Sulpirid, Sultamicillin, Sultiam, Sumatriptan, Suxamethoniumchlorid, Tacrin, Tacrolimus, Taliolol, Tamoxifen, Taurolidin, Tazaroten, Temazepam, Teniposid, Tenoxicam, Terazosin, Terbinafin, Terbutalin, Terfenadin, Terlipressin, Tertatolol, Tetracycline, Tetryzolin, Theobromin, Theophyllin, Butizin, Thiamazol, Phenothiazine, Thiotepa, Tiagabin, Tiaprid, Propionsaurederivate, Ticlopidin, Timolol, Tinidazol, Tioconazol, Tioguanin, Tioxolon, Tiropramid, Tizanidin, Tolazolin, Tolbutamid, Tolcapon, Tolnaftat, Tolperison, Topotecan, Torasemid, Antiestrogene, Tramadol, Tramazolin, Trandolapril, Tranylcypromin, Trapidil, Trazodon, Triamcinolon und Triamcinolonderivate, Triamteren, Trifluperidol, Trifluridin, Trimethoprim, Trimipramin, Tripelennamin, Triprolidin, Trifosfamid, Tromantadin, Trometamol, Tropalpin, Troxerutin, Tulobuterol, Tyramin, Tyrothricin, Urapidil, Ursodeoxycholsäure, Chenodeoxycholsäure, Valaciclovir, Valproinsäure, Vancomycin, Vecuroniumchlorid, Viagra, Venlafaxin, Verapamil, Vidarabin, Vigabatrin, Viloxazin, Vinblastin, Vincamin, Vincristin, Vindesin, Vinorelbin, Vinpocetin, Viquidil, Warfarin, Xantinolnicotinat, Xipamid, Zafirlukast, Zalcitabin, Zidovudin, Zolmitriptan, Zolpidem, Zoplicon, Zotepin und dergleichen.

Die Wirkstoffe können gewünschtenfalls auch in Form ihrer pharmazeutisch annehmbaren Salze oder Derivate verwendet werden, und im Falle chiraler Wirkstoffe können sowohl optisch aktive Isomere als auch Racemate oder Diastereoisomerengemische eingesetzt werden. Gewünschtenfalls können die erfindungsgemässen Zusammensetzungen auch zwei oder mehrere pharmazeutische Wirkstoffe enthalten.

Als Wirkstoffe, die geeignet sind zur Therapie von Colitis ulcerosa und Morbus Crohn sind insbesondere solche zu nennen, die im Darm, insbesondere kurz vor oder erst im Dickdarmbereich möglichst konstant freigesetzt werden sollen. Der pharmazeutische Wirkstoff kann ein Aminosalicylat, ein Sulfonamid oder ein Glucocorticoid sein, insbesondere sind 5-Aminosalicylsäure, Olsalazin, Sulfalazin, Prednison oder Budesonid zu nennen.

Die folgende Tabelle faßt geeignete Wirkstoffe zur Therapie von Colitis ulcerosa und Morbus Crohn zusammen.

### Wirksoffe für die Therapie der Colitis ulcerosa

Mesalazin
Sulfasalazin
Bethamethason-21-dihydrogenophosphat
Hydrocortison-21-acetat
Cromoglicinsäure
Dexamethason
Olsalazin-Na
Budesonid
Bismunitrat, Karaya Gummi
Methylprednisolon-21-hydrogensuccinat
Prednison
Myhrre, Kaffekohle, Kamillenblüttenextrakt
10% Suspension von Humanplacenta

### Weitere geeignete Wirkstoffe

Balsalazid
Oral verabreichte Peptide (z.B. RDP 58)
Interleukin 6
Interleukin 12
Ilodecakin (Interleukin 10)
Nicotintartrat
5-ASA Konjugate (CPR 2015)
Monoclonaler Antikörper gegen Interleukin 12
Diethyldihydroxyhomospermin (DEHOHO)
Diethylhomospermin (DEHOP)
Cholecystokinin (CCK) Antagonist (CR 1795)
15 Aminosäure-Fragment eines 40 kd Peptids aus Magensaft (BPC 15)
Glucocorticoidanalogon (CBP 1011)
Natalizumab
Infliximab (REMICADE)
N-de-Acetyliertes Lysoglycosphingolipid (WILD 20)
Azelastine
Tranilast
Sudismase
Phosphorothioat Antisensoligonucleotid (ISIS 2302)
Tazofelone
Ropivacaine
5 Lipoxygenaseinhibitor (A 69412)
Sucralfat

### Applikationsformen

Die beschriebene (orale) Arzneiform kann als Tablette aus verpreßten Pellets oder in Form von Pellets vorliegen, die in eine Kapsel, z. B. aus Gelatine, Stärke oder Cellulosederivaten, eingefüllt sind.

### Pharmazeutisch übliche Hilfsstoffe

Bei der Herstellung der Arzneiform können pharmazeutisch übliche Hilfsstoffe in an sich bekannter Weise eingesetzt werden. Diese Hilfsstoffe können im Kern oder im Überzugsmittel enthalten sein.

Trockenstellmittel (Antihaftmittel): Trockenstellmittel haben folgende Eigenschaften: sie verfügen über große spezifische Oberflächen, sind chemisch inert, sind gut rieselfähig und feinteilig. Aufgrund dieser Eigenschaften erniedrigen sie die Klebrigkeit von Polymeren, die als funktionelle Gruppen polare Comonomere enthalten.
Beispiele für Trockenstellmittel sind:
Aluminiumoxid, Magnesiumoxid, Kaolin, Talkum, Kieselsäure (Aerosile), Bariumsulfat und Cellulose.

### Trennmittel

Beispiele für Trennmittel sind:
Ester von Fettsäuren oder Fettsäureamide , aliphatische, langkettige Carbonsäuren, Fettalkohole sowie deren Ester, Montan- oder Paraffinwachse und Metallseifen, insbesondere zu nennen sind Glycerolmonostearat, Stearylalkohol, Glycerolbehensäureester, Cetylalkohol, Palmitinsäure, Kanaubawachs, Bienenwachs etc.. Übliche Mengenanteile liegen im Bereich von 0,05 Gew-% bis 5, bevorzugt 0,1 bis 3 Gew.-% bezogen auf das Copolymere.

Weitere pharmazeutisch übliche Hilfsstoffe: Hier sind z. B, Stabilisatoren, Farbstoffe, Antioxidantien, Netzmittel, Pigmente, Glanzmittel etc. zu nennen. Sie dienen vor allem als Verarbeitungshilfsmittel und sollen ein sicheres und reproduzierbares Herstellungsverfahren sowie gute Langzeitlagerstabilität gewährleisten werden kann. Weitere pharmazeutisch übliche Hilfsstoffe können in Mengen von 0,001 Gew-% bis 100 Gew.-%, bevorzugt 0,1 bis 10 Gew.-% bezogen auf den Polymerüberzug vorliegen.

Weichmacher: Als Weichmacher geeignete Stoffe haben in der Regel ein Molekulargewicht zwischen 100 und 20 000 und enthalten eine oder mehrere hydrophile Gruppen im Molekül, z. B. Hydroxyl- , Ester- oder Aminogruppen. Geeignet sind Citrate, Phthalate, Sebacate, Rizinusöl. Beispiele geeigneter Weichmacher sind Citronensäurealkylester, Glycerinester, Phthalsäurealkylester, Sebacinsäurealkylester, Sucroseester, Sorbitanester, Dibutylsebacat und Polyethylenglykole 4000 bis 20.000. Bevorzugte Weichmacher sind Tributylcitrat, Triethylcitrat, Acetyltriethylcitrat, Dibutylsebacat und Diethylsebacat. Die Einsatzmengen liegen zwischen 1 und 35, bevorzugt 2 bis 10 Gew.-% .%, bezogen auf das (Meth)acrylatCopolymere.

### Wirkstoffhaltige Pellets

Wirkstoffhaltige Pellets können hergestellt werden indem man mittels eines Layeringprozesses Wirkstoff aufbringt. Dazu wird Wirkstoff gemeinsam mit weiteren Hilfsstoffen (Trennmittel, ggf. Weichmacher) homogenisiert und in einem Bindemittel (z.B. EUDRAGIT L 30 D-55) gelöst oder suspendiert. Mittels eines Wirbelschichtverfahrens kann die Flüssigkeit auf Placebopellets oder sonstige geeignete Trägermaterialien aufgebracht werden, wobei das Lösungs- oder Suspendiermittel verdunstet wird (Literatur: International Journal of Pharmaceutics 143, S. 13 - 23). Nach dem Herstellverfahren kann sich ein Trocknungsschritt anschließen. Der Wirkstoff kann in mehreren Schichten aufgebracht werden.

Alternativ können wirkstoffhaltige Pellets über ein Extrusions-SphäronisationsVerfahren hergestellt werden. Dies kann z.B. wie folgt ausgeführt werden: Lactose (20%) und Wirkstoff (80%; Mesalazine = 5-ASA) wurden in einem Hochgeschwindigkeits-Mischer (High Speed Mixer, DIOSNA Typ P10, Osnabrück, Germany) gemischt und eine wäßrige Lösung enthaltend den Hilfsstoff Kollidon 25 wurde in kleinen Mengen zugegeben, bis eine homogene Masse erhalten wurde. Die feuchte Pulvermischung wurde gesiebt. Anschließend wurden daraus Pellets mit Hilfe eines Spheronizer Typ 15 (Caleva, Ascot, UK) geformt.

Die Beschichtung mit dem FS-Polymer erfolgte in einem Glatt-Coater (Typ WSG5 oder GPCG1, Glatt GmbH, Binzen/Lörrach, Germany). Es wurde eine 20 %-ige Schicht (bezogen auf Trockengewicht) auf die Pellets mit der Top-Spray Methode in an sich üblicher Weise aufgebracht.

Einige Wirkstoffe, z. B. Acetylsalicylsäure, sind in Form von Wirkstoffkristallen handelsüblich und können in dieser Form anstelle von wirkstoffhaltigen Pellets eingesetzt werden.

Filmüberzüge auf wirkstoffhaltige Pellets werden üblicherweise in Wirbelschichtgeräten aufgebracht. Rezepturbeispiele sind in dieser Anmeldung erwähnt. Filmbildner werden üblicherweise mit Weichmacher und Trennmittel nach einem geeigneten Verfahren gemischt. Hierbei können die Filmbildner als Lösung oder Suspension vorliegen. Die Hilfsstoffe für die Filmbildung können ebenfalls gelöst oder suspendiert sein. Organische oder wässrige Löse- oder Dispergiermittel können verwendet werden. Zur Stabilisierung der Dispersion können zusätzlich Stabilisatoren verwendet werden (Beispiel: Tween 80 oder andere geeignete Emulgatoren bzw. Stabilisatoren).

Beispiele für Trennmittel sind Glycerolmonostearat oder andere geeignete Fettsäurederivate, Kieselsäurederivate oder Talkum. Beispiele für Weichmacher sind Propylenglykol, Phthalate, Polyethylenglykole, Sebacate oder Citrate, sowie andere in der Literatur erwähnte Substanzen.

Allgemeine Bedingungen der Freisetzungstests (z.B. USP 23): pH 1,2: Simulierter Magensaft ohne Pepsin (SGF-sp), pH 6,8 und pH 7,2: Phosphatpuffer nach DAB 10. ERWEKA Typ DT 80 Apparatur "(Paddle); 900 ml Testmedium bei 37 °C, 100 Upm. Die Versuche wurden jeweils dreifach durchgeführt.

### Herstellung multipartikulärer Arzneiformen

Die Herstellung der multipartikulären Arzneiform erfolgt durch Mischen der unterschiedlichen Pelletformen A und B, je nach enthaltener Wirkstoffmenge z. B. in Verhältnis 1 : 1 oder einem anderen Verhältnis, Einfüllen in eine Kapsel oder durch Verpressen zu einer Tabletteneinheit in Gegenwart von Hilfsstoffen in die multipartikuläre Arzneiform.

Die Herstellung von multipartikulären Arzneiformen durch Verpressen eines pharmazeutisch üblichen Bindemittels mit wirkstoffhaltigen Partikeln ist z. B. Beckert et al. (1996), "Compression of enteric-coated pellets to disintegrating tablets'", International Journal of Pharmaceutics 143, S. 13 - 23, und in WO 96/01624 ausführlich beschrieben.

Mischungen zur Herstellung von Tabletten aus überzogenen Partikeln werden durch Vermischen der Pellets mit geeigneten Bindemitteln für die Tablettierung, nötigenfalls der Zugabe von zerfallsfördernden Substanzen und nötigenfalls der Zugabe von Schmiermitteln zubereitet. Das Mischen kann in geeigneten Maschinen stattfinden. Ungeeignet sind Mischer, die zu Schäden an den überzogenen Partikeln führen, z. B. Pflugscharmischer. Zur Erzielung geeigneter kurzer Zerfallszeiten kann eine spezielle Reihenfolge bei der Zugabe der Hilfsstoffe zu den überzogenen Partikel erforderlich sein. Durch Vormischung mit der überzogenen Partikel mit dem Schmier- oder Formentrennmittel Magnesiumstearat kann dessen Oberfläche hydrophobisiert und somit Verkleben vermieden werden.

Zum Tablettieren geeignete Mischungen enthalten üblicherweise 3 bis 15 Gew.-% eines Zerfallshilfsmittels, z. B. Kollidon CL und z. B. 0,1 bis 1 Gew.-% eines Schmier- und Formentrennmittels wie Magnesiumstearat. Der Bindemittelanteil bestimmt sich nach dem geforderten Anteil an überzogenen Partikeln.

Typische Bindemittel sind z. B. Cellactose^{®}, mikrokristalline Cellulose, Calciumphosphate, Ludipress^{®}, Lactose oder andere geeignete Zucker, Calciumsulfate oder Stärkederivate. Bevorzugt werden Substanzen mit geringer Schüttdichte.

Typische Zerfallshilfsmittel (Sprengmittel) sind quervernetzte Stärke- oder Cellulosederivate, sowie quervernetztes Polyvinylpyrrolidon. Ebenso sind Cellulosederivate geeignet. Durch Auswahl eines geeigneten Bindemittels kann die Verwendung von Zerfallshilfsmittel entfallen.

Typische Schmier- und Formentrennmittel sind Magnesiumstearate oder andere geeignete Salze von Fettsäuren oder in der Literatur zu diesem Zweck aufgeführte Substanzen (z.B. Laurinsäure, Calciumstearat, Talkum usw.). Bei Verwendung geeigneter Maschinen (z.B. Tablettenpresse mit externer Schmierung) oder geeigneter Formulierungen kann die Verwendung eines Schmier- und Formentrennmittels in der Mischung entfallen.

Der Mischung kann gegebenenfalls ein Hilfsmittel zur Fließverbesserung beigefügt sein (z.B. hochdisperse Kieselsäurederivate, Talkum usw.).

Das Tablettieren kann auf üblichen Tablettenpressen, Exzenter- oder Rundlauftablettenpressen erfolgen, bei Preßkräften im Bereich von 5 bis 40 kN, bevorzugt 10-20 kN. Die Tablettenpressen können mit Systemen zur externen Schmierung ausgestattet sein. Gegebenenfalls kommen spezielle Systeme zur Matrizenbefüllung zum Einsatz, die die Matrizenbefüllung mittels Rührflügeln vermeiden.

Unter der Auftragsmenge versteht man den Anteil der aufgesprühten Trockensubstanz des funktionellen filmbildenden Polymers in Gew.-%. Sie liegt bei über 15 bis 38, besonders bevorzugt 18 bis 36, insbesondere 20 bis 30 Gew.-% bezogen auf das Partikelgewicht.

Unter dem Partikelanteil versteht man den Gewichtsanteil der überzogenen Partikel am Gesamtgewicht der Arzneiform, der verpreßten Tabelle, in Gew.-%. Der Partikelanteil der Arzneiform liegt bei 35 - 90, besonders bevorzugt 40 bis 70 Gew.-%. Partikelanteile von 70 bis 90 Gew.-% lassen sich insbesondere erreichen, wenn man sogenannte weiche Kerne anstelle von Zuckerpellets einsetzt.

### BEISPIELE

### Beispiel 1: Pelletform A, Innerer Polymerüberzug

Handelsübliche Kerne, enthaltend den Wirkstoff 5-Aminosalicylsäure, mit einem Durchmesser im Bereich von 0,8 bis 1,25 mm werden mit einem 12 %-igen Überzug aus einem Copolymer aus 60 Gew.-% Methylmethacrylat, 30 Gew.-% Ethylacrylat und 10 Gew.-% 2-Trimethylammoniumethylmethacrylat-Chlorid (EUDRAGIT® RL) überzogen.

Dazu werden zu 200 g einer 30 %-igen Dispersion des Copolymers (EUDRAGIT® RL 30D) 30 g Talkum, 12 g Triethylcitrat und 268 g Wasser gegeben (Feststoffgehalt 20,4 5). Die Beschichtung der Kerne erfolgt in einem Wirbelschichtgerät (STREA 1, Aeromatic-Fielder AG, Bubendorf , Schweiz) mit einer Düsenanordnung im "Bottom-Spray-Mode" und einem Düsendurchmesser von 0,8 mm und einem Sprühdruck von 1,4 bis 1,5 bar. 500g Pellets, Lufteingangstemperatur 32 - 36 °C, Luftausgangstemperatur 25 - 30 °C, Sprührate 2,4 g/min.

### Beispiel 2: Pelletform A, Äußerer Polymerüberzug

Die überzogenen Kerne aus Beipiel 1 werden mit einem äußeren Polymerüberzug aus einem (Meth)acrylat Copolymere aus 50 Gew.-% Methacrylsäure und 50 Gew.-% Ethylacrylat (EUDRAGIT® L100-55 bzw. Dispersion EUDRAGIT® L 30 D-55).

Dazu werden zu 166 g einer 30 %-igen Dispersion des oben genannten Copolymers (EUDRAGIT® L30D-55) 25 g Talkum, 5 g Triethylcitrat und 204 g Wasser gegeben (Feststoffgehalt 20,4 %). Die Beschichtung der Kerne erfolgt in wie in Beispiel 1 angegeben im Wirbelschichtgerät. Es wird ein 20%-iger Polymerauftrag (Polymertrockensubstanz bezogen auf das überzogene Pellet) aufgesprüht.

### Beipiel 3: Pelletform B

Wirkstoffhaltige Pellets werden wie in Beispiel 1, jedoch mit einem (Meth)acrylat Copolymeren, bestehend aus 25 Gew.-%, Methylmethacrylat, 65 Gew.-% Methylacrylat und 10 Gew.-% Methacrylsäure (EUDRAGIT® FS) überzogen.

Dazu werden zu 166 g einer 30 %-igen Dispersion des oben genannten Copolymers (EUDRAGIT® FS 30 D) 4 g Glycerolmonostearat, 2 g Polysorbat 80, 2,5 g Triethylcitrat und 185 g Wasser gegeben (Feststoffgehalt der Sprühdispersion 20 %). Die Beschichtung der Kerne erfolgt in wie in Beispiel 1 angegeben im Wirbelschichtgerät. Es wird ein 20%-iger Polymerauftrag (Polymertrockensubstanz bezogen auf das überzogene Pellet) aufgesprüht.

### Beispiel 4: Rezeptur für eine multipartikuläre Arzneiform aus den Pelletformen A und B gemäß den Beispielen 2 und 3.

| Tablettenrezeptur | |
|---|---|
| Pelletform A | 250,0 g |
| Pelletform B | 250,0 g |
| Cellactose | 417,5 g |
| Kollidon CL | 80,0 g |
| Magnesiumstearat | 2,5 g |

Die Mischung kann direkt auf einer geeigneten Tablettenpresse unter Anwendung von z.B. 15 kN Preßkraft zu Tabletten verpreßt werden.

## Patentansprüche

1. Multipartikuläre Arzneiform, geeignet zur gleichmäßigen Freisetzung eines pharmazeutischen Wirkstoffs im Dünndarm und im Dickdarm, enthaltend mindestens zwei Formen von Pellets A und B, die im Kern einen pharmazeutischen Wirkstoff enthalten und unterschiedliche Polymerüberzüge aufweisen, die die Freisetzung des Wirkstoffs bei unterschiedlichen pH-Werten bestimmen, **dadurch gekennzeichnet, dass**
die Pelletform A mit einem inneren Polymerüberzug versehen ist, der eine kontinuieniche Wirkstofffreigabe ermöglicht, und einen äußeren magensaftresistenten Überzug aufweist, der sich oberhalb von etwa pH 5,5 schnell auflöst und der aus einem (Meth)acrylat-Copolymer aus 40 bis 60 Gew.-% Methacrylsäure und 60 bis 40 Gew.% Methylmethacrylat oder 60 bis 40 Gew.-% Ethylacrylat, oder HPMCP (Hydroxypropylmethylcellulosephthalat), besteht und
die Pelletform B mit einem Polymerüberzüg versehen ist, der im Freisetzungstest nach USP bei pH 6,8 in 6 Stunden weniger als 20 % des Wirkstoff freisetzt und bei pH 7,2 in 6 Stunden mehr als 50 % des Wirkstoffs freisetzt, wobei der Polymerüberzug der Pelletform B ein (Meth)acrylat-Copolymer ist, das sich aus 60 bis 95 Gew.-% radikalisch polymerisierten C₁- bis C₄-Alkylestern der Acryl- oder der Methacrylsäure und 5 bis 40 Gew.-% (Meth)acrylatMonomeren mit einer Säuregruppe im Alkylrest zusammensetzt.

2. Multipartikuläre Arzneiform nach Anspruch 1, **dadurch gekennzeichnet, dass** der innere Polymerüberzug der Pelletform A aus einem (Meth)acrylat-Copolymer, aus radikalisch polymerisierten C1- bis C4-Alkylestern der Acryl- oder der Methacrylsäure und (Meth)acrylatMonomeren mit einer quaternären Ammoniumgruppe im Alkylrest, einem (Meth)acrylat Copolymeren aus 20 bis 40 Gew.-% Ethylacrylat und 60 bis 80 Gew.-% Methylmethacrylat, Ethylcellulose oder Polyvinylacetat besteht.

3. Multipartikuläre Arzneiform nach Anspruch 2, **dadurch gekennzeichnet, dass** für den inneren Polymerüberzug der Pelletform A ein (Meth)acrylat-Copolymer aus 85 bis weniger als 93 Gew.-% C1- bis C4-Alkylestern der Acryl- oder der Methacrylsäure und mehr als 7 bis 15 Gew.-% (Meth)acrylat Monomeren mit einer quaternären Ammoniumgruppe im Alkylrest eingesetzt wird.

4. Multipartikuläre Arzneiform nach Anspruch 1, **dadurch gekennzeichnet, dass** für den Polymerüberzug der Pelletform B ein (Meth)acrylat Copolymer, bestehend aus 10 bis 30 Gew.-%, Methylmethacrylat, 50 bis 70 Gew.-% Methylacrylat und 5 bis 15 Gew.-% Methacrylsäure eingesetzt wird.

5. Multipartikuläre Arzneiform nach Anspruch 1 oder 4, **dadurch gekennzeichnet, dass** die Pelletform B zusätzlich mit einem inneren Polymerüberzug versehen ist, der eine kontinuierliche Wirkstofffreigabe ermöglicht.

6. Multipartikuläre Arzneiform nach einem oder mehreren der Ansprüche 1 bis 5, **dadurch gekennzeichnet, dass** der enthaltene pharmazeutische Wirkstoff ein Aminosalicylat, ein Sulfonamid, ein Hormon, ein Peptid, ein Interferon oder ein Glucocorticoid ist.

7. Multipartikuläre Arzneiform nach Anspruch 6, **dadurch gekennzeichnet, dass** der pharmazeutische Wirkstoff 5-Aminosalicylsäure, Olsalazin, Sulfalazin, Prednison oder Budesonid ist.

8. Verfahren zur Herstellung einer multipartikulären Arzneiform nach einem oder mehreren der Ansprüche 1 bis 7, **dadurch gekennzeichnet, dass** man die unterschiedlichen Pelletformen A und B mittels Überziehens von wirkstoffhaltigen Kernen mit den angegebenen Polymerüberzügen herstellt, miteinander mischt und durch Einfüllen in eine Kapsel oder Verpressen zu einer Tabletteneinheit in Gegenwart von Hilfsstoffen in eine multipartikuläre Arzneiform überführt.

9. Verwendung der Pelletformen A und B gemäß einem oder mehreren der Ansprüche 1 bis 7 in einem Verfahren nach Anspruch 8 zur Herstellung einer multipartikulären Arzneiform mit einer gleichmäßigen Wirkstoffabgabe im pH-Bereich von 6,8 und 7,2, entsprechend den Verhältnissen in Dünn- und Dickdarm.

10. Verwendung nach Anspruch 9, **dadurch gekennzeichnet, dass** die multipartikuläre Arzneiform zur Behandlung von Morbus Crohn oder Colitis ulcerosa geeignet ist.

## Claims

1. Multiparticulate drug form suitable for uniform release of an active pharmaceutical ingredient in the small intestine and in the large intestine, comprising at least two forms of pellets A and B which comprise an active pharmaceutical ingredient in the core and have different polymer coatings which determine the release of the active ingredient at different pH values, **characterized in that**
pellet form A is provided with an inner polymer coating which enables continuous release of active ingredient, and has an outer enteric coating which rapidly dissolves above about pH 5.5, and which consists of a (meth)acrylate copolymer of 40 to 60% by weight methacrylic acid and 60 to 40% by weight methyl methacrylate or 60 to 40% by weight ethyl acrylate or HPMCP (hydroxypropylmethylcellulose phthalate), and
pellet form B is provided with a polymer coating which, in the USP release test, releases less than 20% of the active ingredient at pH 6.8 in 6 hours and releases more than 50% of the active ingredient at pH 7.2 in 6 hours, wherein the polymer coating of pellet form B is a (meth)acrylate copolymer which is composed of 60 to 95% by weight free-radical polymerized C₁- to C₄-alkyl esters of acrylic or methacrylic acid and 5 to 40% by weight (meth)acrylate monomers with an acidic group in the alkyl radical.

2. Multiparticulate drug form according to Claim 1, **characterized in that** the inner polymer coating of pellet form A consists of a (meth)acrylate copolymer, of free-radical polymerized C1- to C4-alkyl esters of acrylic or methacrylic acid and (meth)acrylate monomers with a quaternary ammonium group in the alkyl radical, a (meth)acrylate copolymer of 20 to 40% by weight ethyl acrylate and 60 to 80% by weight methyl methacrylate, ethylcellulose or polyvinyl acetate.

3. Multiparticulate drug form according to Claim 2, **characterized in that** a (meth)acrylate copolymer of 85 to less than 93% by weight C1- to C4-alkyl esters of acrylic or methacrylic acid and more than 7 to 15% by weight (meth)acrylate monomers with a quaternary ammonium group in the alkyl radical is employed for the inner polymer coating of pellet form A.

4. Multiparticulate drug form according to Claim 1, **characterized in that** a (meth)acrylate copolymer consisting of 10 to 30% by weight methyl methacrylate, 50 to 70% by weight methyl acrylate and 5 to 15% by weight methacrylic acid is employed for the polymer coating of pellet form B.

5. Multiparticulate drug form according to Claim 1 or 4, **characterized in that** pellet form B is additionally provided with an inner polymer coating which enables continuous release of active ingredient.

6. Multiparticulate drug form according to one or more of Claims 1 to 5, **characterized in that** the active pharmaceutical ingredient which is present is an aminosalicylate, a sulphonamide, a hormone, a peptide, an interferon or a glucocorticoid.

7. Multiparticulate drug form according to Claim 6, **characterized in that** the active pharmaceutical ingredient is 5-aminosalicylic acid, olsalazine, sulfasalazine, prednisone or budesonide.

8. Process for producing a multiparticulate drug form according to one or more of Claims 1 to 7, **characterized in that** the different pellet forms A and B are produced by coating active ingredient-containing cores with the indicated polymer coatings, are mixed together and are converted into a multiparticulate drug form by introduction into a capsule or compression to a tablet unit in the presence of excipients.

9. Use of pellet forms A and B according to one or more of Claims 1 to 7 in a process according to Claim 8 for producing a multiparticulate drug form with uniform release of active ingredient in the pH region of 6.8 and 7.2, corresponding to the conditions in the small and large intestine.

10. Use according to Claim 9, **characterized in that** the multiparticulate drug form is suitable for the treatment of Crohn's disease or ulcerative colitis.

## Revendications

1. Forme galénique multiparticulaire convenant pour la libération régulière d'un principe actif pharmaceutique dans l'intestin grêle et dans le côlon, contenant au moins deux formes de pellets A et B qui contiennent dans le noyau un principe actif pharmaceutique et présentent différents revêtements de polymère qui déterminent la libération du principe actif en cas de valeurs de pH différentes, **caractérisée en ce que**
la forme de pellet A est dotée d'un revêtement de polymère interne qui permet une libération continue du principe actif et présente un revêtement extérieur résistant aux sucs gastriques qui se dissout rapidement au-delà d'un pH d'environ 5,5 et se compose d'un copolymère de (méth)acrylate de 40 à 60 % en poids d'acide méthacrylique et 60 à 40% en poids de méthylméthacrylate ou 60 à 40% en poids d'éthylacrylate ou de HPMCP (phthalate d'hydroxypropylméthylcellulose) et
la forme de pellet B est dotée d'un revêtement de polymère qui libère moins de 20% du principe actif en 6 heures à un pH de 6,8 dans le test de libération selon USP et libère plus de 50% du principe actif en 6 heures à un pH de 7,2, le revêtement polymère de la forme de pellet B étant un copolymère de (méth)acrylate qui se compose de 60 à 95% en poids de C₁- à C₄-alkylesters de l'acide acrylique ou méthacrylique, polymérisés radicalairement, et 5 à 40% en poids de monomères de (méth)acrylate avec un groupe acide dans le radical alkyle.

2. Forme galénique multiparticulaire selon la revendication 1, **caractérisée en ce que** le revêtement interne de polymère de la forme de pellet A se compose d'un copolymère de (méth)acrylate de C₁- à C₄-alkylesters de l'acide acrylique ou méthacrylique, polymérisés radicalairement et de monomères de (méth)acrylate avec un groupe ammonium quaternaire dans le radical alkyle, un copolymère de (méth)acrylate de 20 à 40% en poids d'éthylacrylate et 60 à 80% en poids de méthylméthacrylate, d'éthylcellulose ou de polyvinylacétate.

3. Forme galénique multiparticulaire selon la revendication 2, **caractérisée en ce que**, pour le revêtement interne de polymère de la forme de pellet A, on utilise un copolymère de (méth)acrylate de 85 à moins de 93% en poids de C₁-C₄-alkylesters de l'acide acrylique ou méthacrylique et de plus de 7 à 15% en poids de monomères de (méth)acrylate avec un groupe ammonium quaternaire dans le radical alkyle.

4. Forme galénique multiparticulaire selon la revendication 1, **caractérisée en ce que**, pour le revêtement polymère de la forme de pellet B, on utilise un copolymère de (méth)acrylate composé de 10 à 30% en poids de méthylméthacrylate, 50 à 70% en poids de méthylacrylate et 5 à 15% en poids d'acide méthacrylique.

5. Forme galénique multiparticulaire selon la revendication 1 ou 4, **caractérisée en ce que** la forme de pellet B est dotée en plus d'un revêtement de polymère interne qui permet une libération continue des principes actifs.

6. Forme galénique multiparticulaire selon une ou plusieurs des revendications 1 à 5, **caractérisée en ce que** le principe actif pharmaceutique contenu est un aminosalicylate, un sulfonamide, une hormone, un peptide, un interféron ou un glucocorticoïde.

7. Forme galénique multiparticulaire selon la revendication 6, **caractérisée en ce que** le principe actif pharmaceutique est l'acide 5-aminosalicylique, l'olsalazine, la sulfasalazine, la prednisone ou le budésonide.

8. Procédé de préparation d'une forme galénique multiparticulaire selon une ou plusieurs des revendications 1 à 7, **caractérisé en ce que** l'on prépare les différentes formes de pellet A et B à l'aide du revêtement de noyaux contenant le principe actif avec les revêtements de polymère indiqués, on les mélange l'un à l'autre et on les transforme par remplissage d'une gélule ou compression en comprimé en présence d'adjuvants en une forme galénique multiparticulaire.

9. Utilisation des formes de pellet A et B selon une ou plusieurs des revendications 1 à 7 dans un procédé selon la revendication 8 pour la préparation d'une forme galénique multiparticulaire avec une délivrance régulière du principe actif dans une gamme de pH de 6,8 et 7,2 correspondant aux conditions dans l'intestin grêle et le côlon.

10. Utilisation selon la revendication 9, **caractérisée en ce que** la forme galénique multiparticulaire convient pour le traitement de la maladie de Crohn ou de la colite ulcéreuse.
